# NEW EUROPEAN PATENT SPECIFICATION

(11) **EP 1 711 481 B2**
(45) Date of publication and mention of the opposition decision: **22.05.2013**
(45) Mention of the grant of the patent: 30.12.2009
(21) Application number: 05722772.0
(22) Date of filing: 04.02.2005
(51) Int. Cl.: A61K 31/506

(54) **CRYSTALLINE MONOHYDRATE AS KINASE INHIBITORS**
KRISTALLINES MONOHYDRAT ALS KINASEINHIBITOR
MONOHYDRATE CRISTALLINE COMME INHIBITEURS DE KINASES

(30) Priority: 06.02.2004 US 542490 P; 04.11.2004 US 624937 P
(43) Date of publication of application: 18.10.2006
(73) Proprietor: Bristol-Myers Squibb Company, Princeton, NJ 08543-4000 (US)
(72) Inventor: CHEN, Bang-Chi, Plainsboro, NJ 08536 (US); DROGHINI, Roberto, Candiac, Quebec J5R 3T5 (CA); LAJEUNESSE, Jean, Candiac, Quebec J5R 4T2 (CA); DIMARCO, John, D., East Brunswick, NJ 08816 (US); GALELLA, Michael, Kendall Park, NJ 08824 (US); CHIDAMBARAM, Ramakrishnan, Pennington, NJ 08534 (US)
(74) Representative: Beacham, Annabel Rose
(86) International application number: PCT/US2005/003728
(87) International publication number: WO 2005/077945

(56) References cited:
- EP-A- 0 639 574
- WO-A-2004/071440
- WO-A-2005/072826
- US-A- 3 547 917
- US-B1- 6 596 746
- ZHAO R ET AL: "A new facile synthesis of 2-aminothiazole-5-carboxylates" TETRAHEDRON LETTERS, ELSEVIER SCIENCE PUBLISHERS, AMSTERDAM, NL, vol. 42, no. 11, 11 March 2001 (2001-03-11), pages 2101-2102, XP004229211 ISSN: 0040-4039
- SHAH NEIL P ET AL: "Overriding imatinib resistance with a novel ABL kinase inhibitor." SCIENCE. 16 JUL 2004, vol. 305, no. 5682, 16 July 2004 (2004-07-16), pages 399-401, XP002361026 ISSN: 1095-9203

## Description

### FIELD OF THE INVENTION

The present invention relates to a crystalline form of compound (IV) which is useful as a kinase inhibitor, such as an inhibitor of protein tyrosine kinase and p38 kinase.

### BACKGROUND OF THE INVENTION

Aminothiazole-aromatic amides of formula I 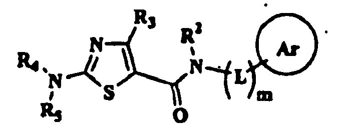 wherein Ar is aryl or heteroaryl, L is an optional alkylene linker, and R₂, R₃, R₄, and R₅, are as defined in the specification herein, are useful as kinase inhibitors; in particular, inhibitors of protein tyrosine kinase and p38 kinase. They are expected to be useful in the treatment of protein tyrosine kinase-associated disorders such as immunologic and oncological disorders [*see,* US Pat. No. 6,596,746 (the '746 patent), assigned to the present assignee and incorporated herein by reference], and p38 kinase-associated conditions such as inflammatory and immune conditions, as described in US patent application Serial No. 10/773,790, filed February 6, 2004, claiming priority to US Provisional application Serial No. 60/445,410, filed February 6, 2003 (hereinafter the '410 application), both of which are also assigned to the present assignee and incorporated herein by reference.

The compound of formula (IV), 'N-(2-Chloro-6-methylphenyl)-2-[[6-[4-(2-hydroxyethyl)-1-piperazinyl]-2-methyl-4-pyrimidinyl]amino]-5-thiazolecarboxamide, is an inhibitor of SRC/ABL and is useful in the treatment of oncological diseases.

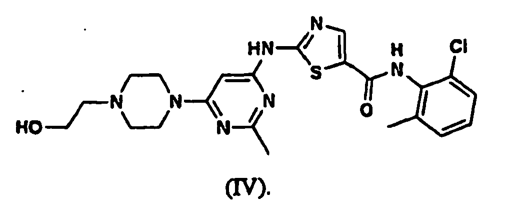

Other approaches to preparing 2-aminothiazole-5-carboxamides are described in the '746 patent and in the '410 application. The '746 patent describes a process involving treatment of chlorothiazole with n-BuLi followed by reaction with phenyl isocyanates to give chlorothiazole-benzamides, which are further elaborated to aminothiazole-benzamide final products after protection, chloro-to-amino substitution, and deprotection, *e.g.*,

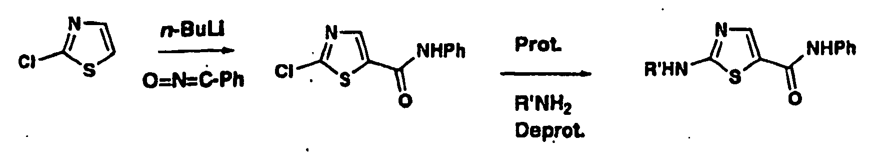

The '410 application describes a multi-step process involving first, converting N-unsubstituted aminothiazole carboxylic acid methyl or ethyl esters to bromothiazole carboxylic acid esters via diazotization with tert-butyl nitrite and subsequent CuBr₂ treatment, *e.g.*, 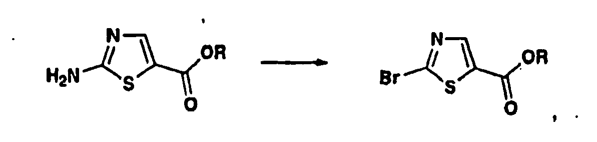 then, hydrolyzing the resulting bromothiazole esters to the corresponding carboxylic acids and converting the acids to the corresponding acyl chlorides, *e.g.*, 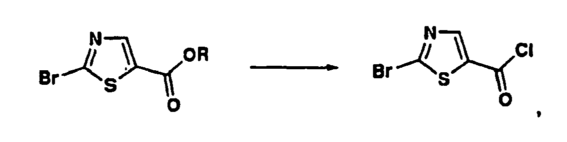 then finally, coupling the acyl chlorides with anilines to afford bromothiazole-benzamide intermediates which were further elaborated to aminothiazole-benzamide final products, *e.g.*,

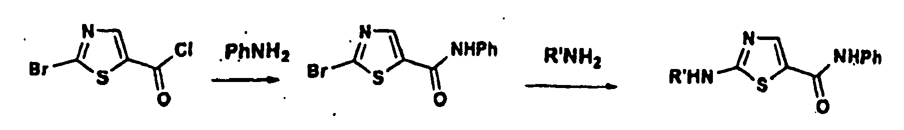

Other approaches for making 2-aminothiazole-5-carboxamides include coupling of 2-aminothiazole-5-carboxylic acids with amines using various coupling conditions such as DCC [Roberts et al, J. Med. Chem. (1972), 15, at p. 1310], and DPPA [Marsham et al., J. Med. Chem. (1991), 34, at p. 1594)].

The above methods present drawbacks with respect to the production of side products, the use of expensive coupling reagents, less than desirable yields, and the need for multiple reaction steps to achieve the 2-aminothiazole-5-carboxamide compounds.

Reaction of N,N-dimethyl-N'-(aminothiocarbonyl)-formamidines with α-haloketones and esters to give 5-carbonyl-2-aminothiazoles has been reported. *See* Lin, Y. et al, J. Heterocycl. Chem. (1979), 16, at 1377; Hartmann, H. et al, J. Chem. Soc. Perkin Trans. (2000), 1, at 4316; Noack, A. et al; Tetrahedron (2002), 58, at 2137; Noack, A.; et al.. Angew. Chem. (2001),113, at 3097; and Kantlehner, W. et al., J. Prakt. Chem.lChem.-Ztg. (1996), 338, at 403. Reaction of β-ethoxy acrylates and thioureas to prepare 2-aminothiazole-5-carboxylates also has been reported. See Zhao, R., et al., Tetrahedron Lett. (2001), 42, at 2101. However, electrophilic bromination of acrylanilide and crotonanilide has been known to undergo both aromatic bromination and addition to the α,β-unsaturated carbon-carbon double bonds. *See* Autenrieth, Chem. Ber. (1905), 38, at 2550; Eremeev et al., Chem. Heterocycl. Compd. Engl. Transl. (1984), 20, at 1102.

New and efficient processes for preparing 2-aminothiazole-5-carboxamides are desired.

### SUMMARY OF THE INVENTION

The present invention is directed to crystalline monohydrate of the compound of formula (IV).

### BRIEF DESCRIPTION OF THE DRAWINGS

The invention is illustrated by reference to the accompanying drawings described below.
Figure 1 shows a simulated (bottom) (calculated from atomic coordinates generated at room temperature) and experimental (top) pXRD patterns for crystalline monohydrate of the compound of formula (IV).
Figure 2 shows a DSC and TGA of the of the monohydrate crystalline form of the compound of Formula (IV).
Figure 3 shows a simulated (bottom) (from atomic parameters refined at room temperature) and experimental (top) pXRD patterns for crystalline butanol solvate of the compound of formula (IV).

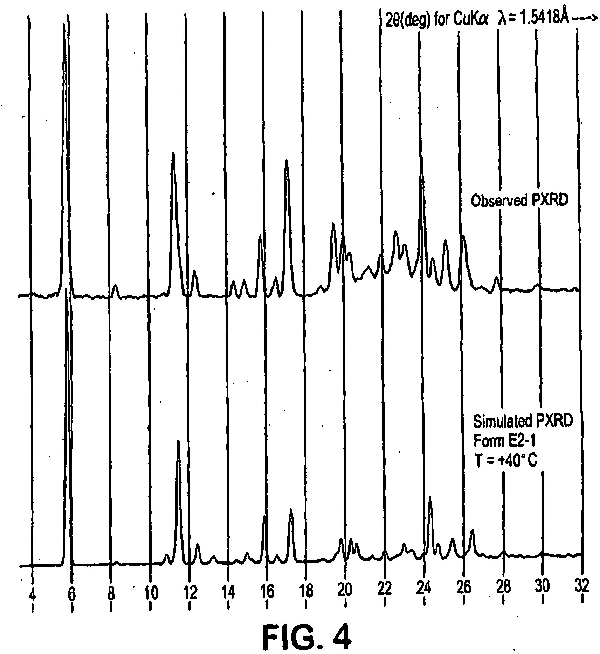

Figure 4 shows a simulated (bottom) (from atomic parameters refined at - 40°C) and experimental (top) pXRD patterns for crystalline ethanol solvate of the compound of formula (IV).

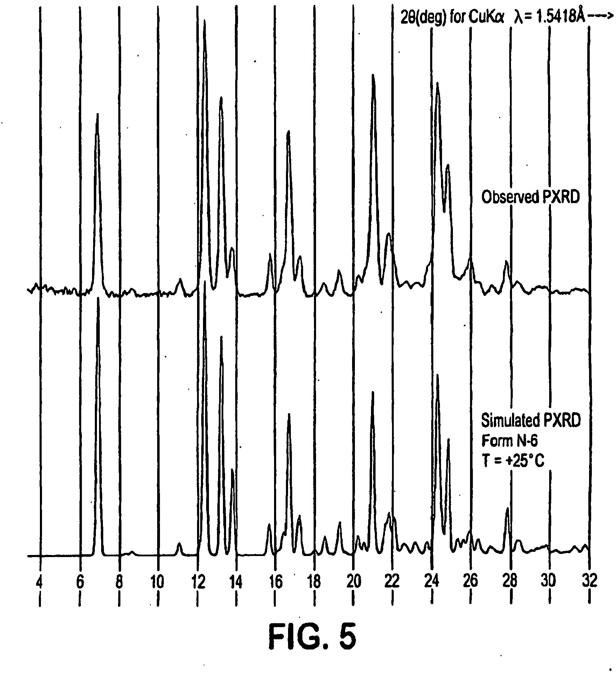

Figure 5 shows a simulated (bottom) (from atomic parameters refined at room temperature) and experimental (top) pXRD patterns for crystalline neat form (N-6) of the compound of formula (IV).

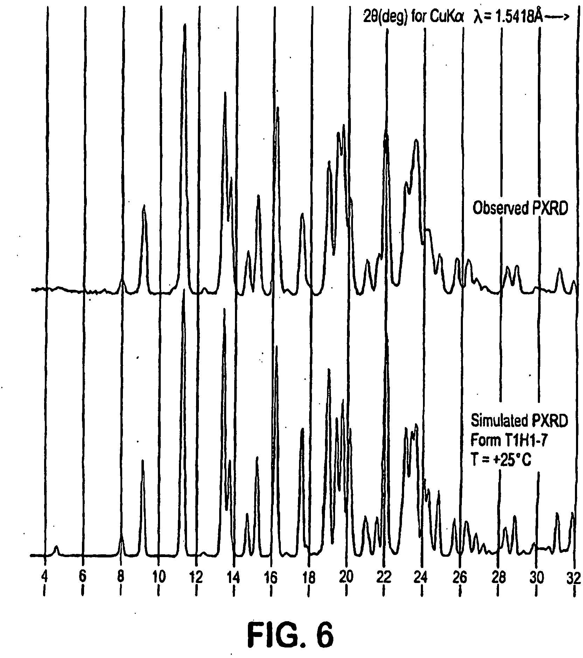

Figure 6 shows a simulated (bottom) (from atomic parameters refined at room temperature) and experimental (top) pXRD patterns for crystalline neat form (T1H1-7) of the compound of formula (IV).

### DETAILED DESCRIPTION OF THE INVENTION

### Abbreviations

For ease of reference, the following abbreviations may be used herein:
Ph = phenyl
Bz = benzyl
t-Bu = tertiary butyl
Me = methyl
Et = ethyl
Pr = propyl
Iso-P = isopropyl
MeOH = methanol
EtOH = ethanol
EtOAc = ethyl acetate
Boc = tert-butyloxycarbonyl
CBZ = carbobenzyloxy or carbobenzoxy or benzyloxycarbonyl
DMF = dimethyl formamide
DMF-DMA = *N,N*-dimethylformamide dimethyl acetal
DMSO = dimethyl sulfoxide
DPPA = diphenylphosphoryl azide
DPPF = 1,1'-bis(diphenylphosphino)ferrocene
HATU = O-benzotriazol-1-y10 N,N,N',N'-tetramethyluronium hexafluorphosphate
LDA = lithium di-isopropyl amide
TEA = triethylamine
TFA = trifluoroacetic acid
THF = tetrahydrofuran
KOH = potassium hydroxide
K₂CO₃ = potassium carbonate
POCl₃ =phosphorous oxychloride
EDC or EDCI = 3-ethyl-3'-(dimethylamino)propyl- carbodiimide
DIPEA = diisopropylethylamine
HOBt= 1-hydroxybenzotriazole hydrate
NBS = N-bromosuccinamide
NMP = N-methyl-2-pyrrolidinone
NaH = sodium hydride
NaOH = sodium hydroxide
Na₂S₂O₃ = sodium thiosulfate
Pd = palladium
Pd-C or Pd/C = palladium on carbon
min = minute(s)
L = liter
mL = milliliter
µL = microliter
g = gram(s)
mg = milligram(s)
mol = moles
mmol = millimole(s)
meq = milliequivalent
RT or rt = room temperature
RBF = round bottom flask
ret. t. = HPLC retention time (minutes)
sat or sat'd = saturated
aq. = aqueous
TLC = thin layer chromatography
HPLC = high performance liquid chromatography
LC/MS = high performance liquid chromatography/mass spectrometry
MS = mass spectrometry
NMR = nuclear magnetic resonance
mp = melting point
DSC = differential scanning calorimetry
TGA = thermogravimetric analysis
XRPD = x-ray powder diffraction pattern
pXRD = x-ray powder diffraction pattern

### Definitions

The following are definitions of terms used in this specification and appended claims. The initial definition provided for a group or term herein applies to that group or term throughout the specification and claims, individually or as part of another group, unless otherwise indicated.

"Suitable solvent" as used herein is intended to refer to a single solvent as well as mixtures of solvents. Solvents may be selected, as appropriate for a given reaction step, from, for example, aprotic polar solvents such as DMF, DMA, DMSO, dimethylpropyleneurea, N-methylpyrrolidone (NMP), and hexamethylphosphoric triamide; ether solvents such as diethyl ether, THF, 1,4-dioxane, methyl t-butyl ether, dimethoxymethane, and ethylene glycol dimethyl ether; alcohol solvents such as MeOH, EtOH, and isopropanol; and halogen-containing solvents such as methylene chloride, chloroform, carbon tetrachloride, and 1,2-dichloroethane. Mixtures of solvents may also include biphasic mixtures.

The term "slurry" as used herein is intended to mean a saturated solution of the compound of Formula (IV) and an additional amount of the compound of Formula (IV) to give a heterogeneous solution of the compound of Formula (IV) and a solvent.

The present invention describes crystalline monohydrate of the compound of formula (IV) in substantially pure form. As used herein, "substantially pure" means a compound having a purity greater than 90 percent, including 90, 91, 92, 93, 94, 95, 96, 97, 98, 99, and 100 percent.

As one example, crystalline monohydrate of the compound of the formula (IV) can be substantially pure in having a purity greater than 90 percent, where the remaining less than 10 percent of material comprises other form(s) of the compound of the formula (IV), and/or reaction and/or processing impurities arising from its preparation. A crystalline monohydrate of the compound of the formula (IV) in substantially pure form may therefore be employed in pharmaceutical compositions to which other desired components are added, for example, excipients, carriers, or active chemical entities of different molecular structure.

When dissolved, crystalline monohydrate of the compound of formula (IV) loses its crystalline structure, and is therefore referred to as a solution of the compound of formula (IV). The monohydrate form of the present invention, however, may be used for the preparation of liquid formulations in which the drug is dissolved or suspended. In addition, the crystalline monohydrate of the compound of formula (IV) may be incorporated into solid formulations.

A therapeutically effective amount of the crystalline monohydrate of the compound of formula (IV) is combined with a pharmaceutically acceptable carrier to produce the pharmaceutical compositions of this invention. By "therapeutically effective amount" it is meant an amount that, when administered alone or an amount when administered with an additional therapeutic agent, is effective to prevent, suppress or ameliorate the disease or condition or the progression of the disease or condition.

In one embodiment, the present invention provides a crystalline monohydrate of the compound of formula (IV).

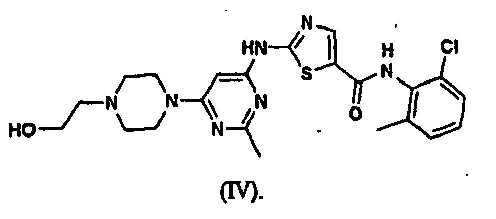

In another embodiment, the monohydrate form is in substantially pure form.

In another embodiment, the monohydrate form is in substantially pure form,
wherein substantially pure is greater than 90 percent pure.

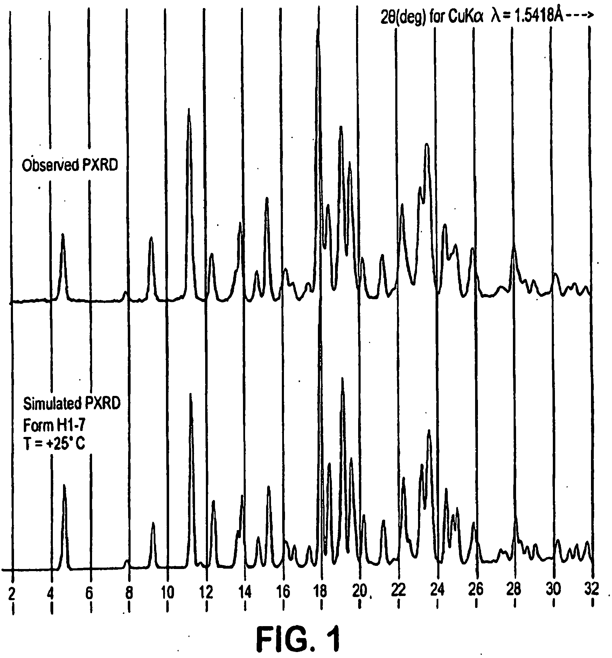

In another embodiment, the monohydrate form of the compound of Formula (IV) is characterized by an x-ray powder diffraction pattern substantially in accordance with that shown in Figure 1.

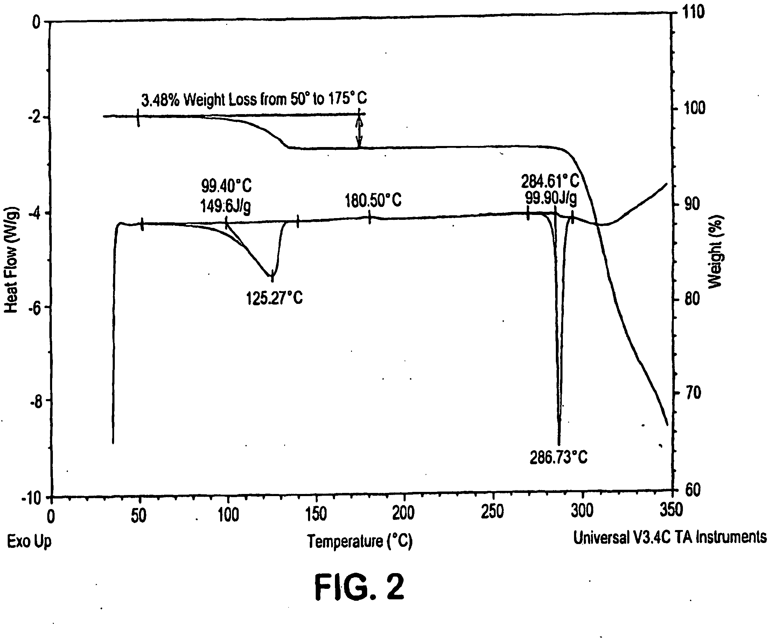

In another embodiment, the monohydrate form of the compound of Formula (IV) is characterized by differential scanning calorimetry thermogram and a thermogravimetric anaylsis substantially in accordance with that shown in Figure 2.

In another embodiment, the monohydrate form of the compound of Formula (IV) is characterized by an x-ray powder diffraction pattern (CuKα λ=1.5418Å at a temperature of about 23°C) comprising four or more 2θ values (alternatively, comprising five or more, six or more, or comprising 2θ values) selected from the group consisting of: 18.0± 0.2,18.4± 0.2, 19.2± 0.2,19.6± 0.2,21.2± 0.2, 24.5± 0.2, 25.9± 0.2, and 28.0± 0.2.

In another embodiment, the monohydrate form of the compound of Formula (IV) is characterized by an x-ray powder diffraction pattern (CuKα λ=1.5418Å at a temperature of about 23 °C) comprising four or more 2θ values (alternatively, comprising five or more, six or more, or comprising 2θ values) selected from the group consisting of: 4.6±0.2,11.2± 0.2,13.8± 0.2,15.2± 0.2,17.9± 0.2, 19.1± 0.2, 19.6± 0.2,23.2± 0.2, 23.6± 0.2.

In another embodiment, the monohydrate form of the compound of Formula (IV) is characterized by unit cell parameters approximately equal to the following:
Cell dimensions: a(Å) = 13.862(1);
b(Å)= 9.286(1);
c(Å) = 38.143(2);
Volume = 4910(1) Å³
Space group Pbca
Molecules/unit cell 8
Density (calculated) (g/cm³) 1.300
   wherein the compound is at a temperature of about -50°C.

In another embodiment, the monohydrate form of the compound of Formula (IV) there is one water molecule per molecule of formula (IV).

The butanol solvate form of the compound of Formula (IV) is characterized by unit cell parameters approximately equal to the following:
Cell dimensions: a(Å)= 22.8102(6);
b(Å)= 8.4691(3);
c(Å)= 15.1436(5);
Volume = 2910.5(2) Å³
Space group P2₁/a
Molecules/unit cell 4
Density (calculated) (g/cm³) 1.283.

The crystalline butanol solvate of the compound of Formula (IV) is characterized by an x-ray powder diffraction pattern (CuKα λ=1.5418Å at a temperature of about 23 °C) comprising four or more 2θ values (alternatively, comprising five or more, six or more, or comprising 2θ values) selected from the group consisting of: 5.9 ± 0.2,12.0 ± 0.2,13.0 ± 0.2,17.7 ± 0.2,24.1 ± 0.2, and 24.6 ± 0.2.

In another embodiment, the present invention describes a pharmaceutical composition comprising a therapeutically effective amount of the crystalline monohydrate of the compound of Formula (IV) and a pharmaceutically acceptable carrier.

In another embodiment, the present invention describes the crystalline monohydrate of the compound of Formula (IV) for use in treating cancer.

In another embodiment, the present invention describes the crystalline monohydrate of the compound of Formula (IV) for use in treating oncological disorders, wherein the disorders are selected from chronic myelogenous leukemia (CML), gastrointestinal stromal tumor (GIST), small cell lung cancer (SCLC), non-small cell lung cancer (NSCLC), ovarian cancer, melanoma, mastocytosis, germ cell tumors, acute myelogenous leukemia (AML), pediatric sarcomas, breast cancer, colorectal cancer, pancreatic cancer, and prostate cancer.

In another embodiment, the present invention is directed to a use of the crystalline monohydrate of the compound of Formula (IV), in the preparation of a medicament for the treatment of oncological disorders, such as those described herein.

In another embodiment, the present invention is directed to the crystalline monohydrate of the compound of Formula (IV) for use in treating oncological disorders, as described herein, which are resistant or intolerant to Gleevec ® (STI-571).

This invention also encompasses all combinations of alternative aspects of the invention noted herein. It is understood that any and all embodiments of the present invention may be taken in conjunction with any other embodiment to describe additional embodiments of the present invention. Furthermore, any elements of an embodiment are meant to be combined with any and all other elements from any of the embodiments to describe additional embodiments.

### UTILITY

The compound of claim 1 prepared according to the invention herein inhibit protein tyrosine kinases, especially Src-family kinases such as Lck, Fyn, Lyn, Src, Yes, Hck, Fgr and Blk, and are thus useful in the treatment, including prevention and therapy, of protein tyrosine kinase-associated disorders such as immunologic and oncologic disorders. The compound of claim 1 also may inhibit receptor tyrosine kinases including HER1 and HER2 and therefore be useful in the treatment of proliferative disorders such as psoriasis and cancer. The ability of these compounds to inhibit HER1 and other receptor kinases will permit their use as anti-angiogenic agents to treat disorders such as cancer and diabetic retinopathy. "Protein tyrosine kinase-associated disorders" are those disorders which result from aberrant tyrosine kinase activity, and/or which are alleviated by the inhibition of one or more of these enzymes. For example, Lck inhibitors are of value in the treatment of a number of such disorders (for example, the treatment of autoimmune diseases), as Lck inhibition blocks T cell activation. The treatment of T cell mediated diseases, including inhibition of T cell activation and proliferation, is a particularly preferred use for compound of claim 1 prepared according to the process herein.

Use of the compound of claim 1 in treating protein tyrosine kinase-associated disorders is exemplified by, but is not limited to, treating a range of disorders such as: transplant (such as organ transplant, acute transplant or heterograft or homograft (such as is employed in burn treatment)) rejection; protection from ischemic or reperfusion injury such as ischemic or reperfusion injury incurred during organ transplantation, myocardial infarction, stroke or other causes; transplantation tolerance induction; arthritis (such as rheumatoid arthritis, psoriatic arthritis or osteoarthritis); multiple sclerosis; chronic obstructive pulmonary disease (COPD), such as emphysema; inflammatory bowel disease, including ulcerative colitis and Crohn's disease; lupus (systemic lupus erythematosis); graft vs. host disease; T-cell mediated hypersensitivity diseases, including contact hypersensitivity, delayed-type hypersensitivity, and gluten-sensitive enteropathy (Celiac disease); psoriasis; contact dermatitis (including that due to poison ivy); Hashimoto's thyroiditis; Sjogren's syndrome; Autoimmune Hyperthyroidism, such as Graves' Disease; Addison's disease (autoimmune disease of the adrenal glands); Autoimmune polyglandular disease (also known as autoimmune polyglandular syndrome); autoimmune alopecia; pernicious anemia; vitiligo; autoimmune hypopituatarism; Guillain-Barre syndrome; other autoimmune diseases; cancers, including cancers where Lck or other Src-family kinases such as Src are activated or overexpressed, such as colon carcinoma and thymoma, and cancers where Src-family kinase activity facilitates tumor growth or survival; glomerulonephritis; serum sickness; uticaria; allergic diseases such as respiratory allergies (asthma, hayfever, allergic rhinitis) or skin allergies; scleracierma; mycosis fungoides; acute inflammatory responses (such as acute respiratory distress syndrome and ishchemia/reperfusion injury); dermatomyositis; alopecia areata; chronic actinic dermatitis; eczema; Behcet's disease; Pustulosis palmoplanteris; Pyoderma gangrenum; Sezary's syndrome; atopic dermatitis; systemic schlerosis; and morphea.

The compound of the present invention is useful for the treatment of cancers such as chronic myelogenous leukemia (CML), gastrointestinal stromal tumor (GIST), small cell lung cancer (SCLC), non-small cell lung cancer (NSCLC), ovarian cancer, melanoma, mastocytosis, germ cell tumors, acute myelogenous leukemia (AML), pediatric sarcomas, breast cancer, colorectal cancer, pancreatic cancer, prostate cancer and others known to be associated with protein tyrosine kinases such as, for example, SRC, BCR-ABL and c-KIT. The compounds of the present invention are also useful in the treatment of cancers that are sensitive to and resistant to chemotherapeutic agents that target BCR-ABL and c-KIT, such as, for example, Gleevec® (STI-571). In one embodiment of the invention, for example, the compound of claim 1 is useful in the treatment of patients resistant or intolerant to Gleevec ® (STI-571) for diseases such as chronic myelogenous leukemias (CML), or other cancers (including other leukemias) as described herein.

In another embodiment of the invention the compound of claim 1 is administered in conjunction with at least one anti-neoplastic agent.

As used herein, the phrase "anti-neoplastic agent" or "anti-cancer agent" is synonymous with "chemotherapeutic agent" and/or "anti-proliferative agent" and refers to compounds that prevent cancer, or hyperproliferative cells from multiplying. Anti-proliferative agents prevent cancer cells from multiplying by: (1) interfering with the cell's ability to replicate DNA and (2) inducing cell death and/or apoptosis in the cancer cells.

Classes of compounds that may be used as anti-proliferative cytotoxic agents and/or anti-proliferative agents include the following:
Alkylating agents (including, without limitation, nitrogen mustards, ethylenimine derivatives, alkyl sulfonates, nitrosoureas and triazenes): Uracil mustard, Chlormethine, Cyclophosphamide (Cytoxan@), Ifosfamide, Melphalan, Chlorambucil, Pipobroman, Triethylene-melamine, Triethylenethiophosphoramine, Busulfan, Carmustine, Lomustine, Streptozocin, Dacarbazine, and Temozolomide.
Antimetabolites (including, without limitation, folic acid antagonists, pyrimidine analogs, purine analogs and adenosine deaminase inhibitors):
   Methotrexate, 5-Fluorouracil, Floxuridine, Cytarabine, 6-Mercaptopurine, 6-Thioguanine, Fludarabine phosphate, Pentostatine, and Gemcitabine.

Natural products and their derivatives (for example, vinca alkaloids, antitumor antibiotics, enzymes, lymphokines and epipodophyllotoxins): Vinblastine, Vincristine, Vindesine, Bleomycin, Dactinomycin, Daunorubicin, Doxorubicin, Epirubicin, Idarubicin, Ara-C, paclitaxel (paclitaxel is commercially available as Taxol®), Mithramycin, Deoxyco-formycin, Mitomycin-C, L-Asparaginase, Interferons (especially IFN-a), Etoposide, and Teniposide.

Other anti-proliferative cytotoxic agents and/or anti-proliferative agents are navelbene, CPT-11, anastrazole, letrazole, capecitabine, reloxafine, cyclophosphamide, ifosamide, and droloxafine.

The phrase "radiation therapy" includes, but is not limited to, x-rays or gamma rays which are delivered from either an externally applied source such as a beam or by implantation of small radioactive sources. Radiation therapy may be useful in combination with compounds of the present invention.

The following may also be useful when administered in combination with compounds of the present invention.

Microtubule affecting agents interfere with cellular mitosis and are well known in the art for their anti-proliferative cytotoxic activity. Microtubule affecting agents useful in the invention include, but are not limited to, allocolchicine (NSC 406042), Halichondrin B (NSC 609395), colchicine (NSC 757), colchicine derivatives (*e.g.*, NSC 33410), dolastatin 10 (NSC 376128), maytansine (NSC 153858), rhizoxin (NSC 332598), paclitaxel (Taxol®, NSC 125973), Taxol® derivatives (*e.g.*, derivatives (*e.g.*, NSC 608832), thiocolchicine NSC 361792), trityl cysteine (NSC 83265), vinblastine sulfate (NSC 49842), vincristine sulfate (NSC 67574), natural and synthetic epothilones including but not limited to epothilone A, epothilone B, epothilone C, epothilone D, desoxyepothilone A, desoxyepothilone B, [1S-[1R*,3R*(E),7R*,10S*,11R*,12R*,16S*]]-7-11-dihydroxy-8,8,10,12,16-pentamethyl-3-[1-methyl-2-(2-methyl-4-thiazolyl)ethenyl]-4-aza-17 oxabicyclo [14.1.0]heptadecane-5,9-dione (disclosed in US Patent 6,262,094, issued July 17, 2001), [1S-[1R*,3R*(E),7R*,10S*,11R*,12R*,16S*]]-3-[2-[2-(aminomethyl)-4-thiazolyl]-1-methylethenyl]-7,11-dihydroxy-8,8,10,12,16-pentamethyl-4-17-dioxabicyclo[14.1.0]- heptadecane-5,9-dione (disclosed in USSN 09/506,481 filed on February 17, 2000, and examples 7 and 8 herein), [1S 1R*,3R*(E),7R*,10S*,11R*,12R*, 16S*]]-7,11-dihydroxy-8,8,10,12,16-pentamethyl-3-[1-methyl- 2-(2-methyl-4-thiazolyl)ethenyl]-4-aza-17oxabicyclo[14.1.0]-heptadecane-5,9-dione, [1S-[1R*,3R*(E),7R*,10S*,11R*,12R*,16S*]]-3-[2-[2-(Aminomethyl)-4-thiazolyl]-1-methylethenyl]-7,11-dihydroxy-8,8,10,12,16-pentamethyl-4,17-dioxabicyclo[14.1.0]heptadecane-5,9-dione, and derivatives thereof; and other microtubule-disruptor agents. Additional antineoplastic agents include, discodermolide (*see* Service, (1996) Science, 274:2009) estramustine, nocodazole, MAP4, and the like. Examples of such agents are also described in the scientific and patent literature, see, *e.g.*, Bulinski (1997) J. Cell Sci. 110:3055 3064; Panda (1997) Proc. Natl. Acad. Sci. USA 94:10560-10564; Muhlradt (1997) Cancer Res. 57:3344-3346; Nicolaou (1997) Nature 387:268-272; Vasquez (1997) Mol. Biol. Cell. 8:973-985; Panda (1996) J. Biol. Chem 271:29807-29812.

In cases where it is desirable to render aberrantly proliferative cells quiescent in conjunction with or prior to treatment with the chemotherapeutic methods of the invention, hormones and steroids (including synthetic analogs): 17a-Ethinylestradiol, Diethylstilbestrol, Testosterone, Prednisone, Fluoxymesterone, Dromostanolone propionate, Testolactone, Megestrolacetate, Methylprednisolone, Methyl-testosterone, Prednisolone, Triamcinolone, hlorotrianisene, Hydroxyprogesterone, Aminoglutethimide, Estramustine, Medroxyprogesteroneacetate, Leuprolide, Flutamide, Toremifene, Zoladex can also be administered to the patient.

Also suitable for use in the combination chemotherapeutic methods of the invention are antiangiogenics such as matrix metalloproteinase inhibitors, and other VEGF inhibitors, such as anti-VEGF antibodies and small molecules such as ZD6474 and SU6668 are also included. Anti- Her2 antibodies from Genetech may also be utilized. A suitable EGFR inhibitor is EKB-569 (an irreversible inhibitor). Also included are Imclone antibody C225 immunospecific for the EGFR, and src inhibitors.

Also suitable for use as an antiproliferative cytostatic agent is Casodex™ which renders androgen-dependent carcinomas non-proliferative. Yet another example of a cytostatic agent is the antiestrogen Tamoxifen which inhibits the proliferation or growth of estrogen dependent breast cancer. Inhibitors of the transduction of cellular proliferative signals are cytostatic agents. Examples are epidermal growth factor inhibitors, Her-2 inhibitors, MEK-1 kinase inhibitors, MAPK kinase inhibitors, PI3 inhibitors, Src kinase inhibitors, and PDGF inhibitors.

As mentioned, certain anti-proliferative agents are anti-angiogenic and antivascular agents and, by interrupting blood flow to solid tumors, render cancer cells quiescent by depriving them of nutrition. Castration, which also renders androgen dependent carcinomas non-proliferative, may also be utilized. Starvation by means other than surgical disruption of blood flow is another example of a cytostatic agent. A particular class of antivascular cytostatic agents is the combretastatins. Other exemplary cytostatic agents include MET kinase inhibitors, MAP kinase inhibitors, inhibitors of non-receptor and receptor tyrosine kinases, inhibitors of integrin signaling, and inhibitors of insulin-like growth factor receptors.

Also suitable are anthracyclines (e.g., daunorubicin, doxorubicin), cytarabine (ara-C; Cytosar-U®); 6-thioguanine (Tabloid®), mitoxantrone (Novantrone®) and etoposide (VePesid®),amsacrine (AMSA), and all-trans retinoic acid (ATRA).

The compound of the present invention may be useful in combination with BCR-ABL inhibitors such as, but not limited to, Gleevec® (imatinib, STI-571) or AMN-107, the compound shown below

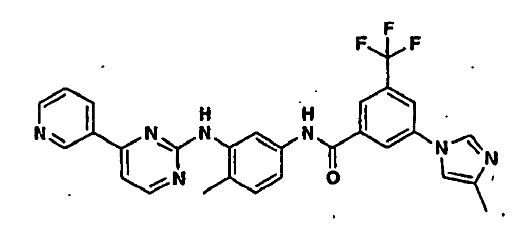

The compound of the present invention may be useful in combination with anti-cancer compounds such as fentanyl, doxorubicin, interferon alfa-n3, palonosetron dolasetron anastrozole, exemestane, bevacizumab, bicalutamide, cisplatin, dacarbazine, cytarabine, clonidine, epirubicin, levamisole, toremifene, fulvestrant, letrozole, tamsulosin, gallium nitrate, trastuzumab, altretamine, hydroxycarbamide, ifosfamide, interferon alfacon-1, gefitinib, granisetron, leuprorelin, dronabinol, megestrol, pethidine, promethazine, morphine, vinorelbine, pegfilgrastim, filgrastim, nilutamide, thiethylperazine, leuprorelin, pegaspargase, muromonab-CD3, porfimer sodium, cisplatin, abarelix, capromab, samarium SM153 lexidronam, paclitaxel, docetaxel, etoposide, triptorelin, valrubicin, nofetumomab merpentan technetium 99m Tc, vincristine, capecitabine, strptozocin, and ondansetron.

Thus, the present invention provides methods for the treatment of a variety of cancers, including, but not limited to, the following:
carcinoma including that of the bladder (including accelerated and metastatic bladder cancer), breast, colon (including colorectal cancer), kidney, liver, lung (including small and non-small cell lung cancer and lung adenocarcinoma), ovary, prostate, testes, genitourinary tract, lymphatic system, rectum, larynx, pancreas (including exocrine pancreatic carcinoma), esophagus, stomach, gall bladder, cervix, thyroid, and skin (including squamous cell carcinoma);
hematopoietic tumors of lymphoid lineage including leukemia, acute lymphocytic leukemia, acute lymphoblastic leukemia, B-cell lymphoma, T-cell lymphoma, Hodgkin's lymphoma, non-Hodgkin's lymphoma, hairy cell lymphoma, histiocytic lymphoma, and Burketts lymphoma;
hematopoietic tumors of myeloid lineage including acute and chronic myelogenous leukemias, myelodysplastic syndrome, myeloid leukemia, and promyelocytic leukemia;
tumors of the central and peripheral nervous system including astrocytoma, neuroblastoma, glioma, and schwannomas;
tumors of mesenchymal origin including fibrosarcoma, rhabdomyoscarcoma, and osteosarcoma; and
other tumors including melanoma, xenoderma pigmentosum, keratoactanthoma, seminoma, thyroid follicular cancer, and teratocarcinoma.

The present invention provides methods for the treatment of a variety of non-cancerous proliferative diseases.

The invention is useful to treat GIST, breast cancer, pancreatic cancer, colon cancer, NSCLC, CML, and ALL, sarcoma, and various pediatric cancers.

The compound of the present invention is a protein tyrosine kinase inhibitor and as such is useful in the treatment of immunological disorders in addition to oncological disorders. U.S. Patent No. 6,596,746 describes the utility of the compound in immunological disorders and is hereby incorporated by reference for the description of the compound in such immunological disorders.

The present invention also encompasses a pharmaceutical composition useful in the treatment of cancer, comprising the administration of a therapeutically effective amount of the combinations of this invention, with or without pharmaceutically acceptable carriers or diluents. The pharmaceutical compositions of this invention comprise an anti-proliferative agent or agents, the claim 1 compound, and a pharmaceutically acceptable carrier. The methods entail the use of a neoplastic agent in combination with the compound of claim 1. The compositions of the present invention may further comprise one or more pharmaceutically acceptable additional ingredient(s) such as alum, stabilizers, antimicrobial agents, buffers, coloring agents, flavoring agents, adjuvants, and the like. The antineoplastic agents, compounds and compositions of the present invention may be administered orally or parenterally including the intravenous, intramuscular, intraperitoneal, subcutaneous, rectal and topical routes of administration.

The present invention also provides using the compound obtained with the inventive process to further prepare pharmaceutical compositions capable of treating Src-kinase associated conditions, including the conditions described above. The said compositions may contain other therapeutic agents. Pharmaceutical compositions may be formulated by employing conventional solid or liquid vehicles or diluents, as well as pharmaceutical additives of a type appropriate to the mode of desired administration (*e.g.*, excipients, binders, preservatives, stabilizers, flavors, etc.) according to techniques such as those well known in the art of pharmaceutical formulations.

The said pharmaceutical compositions may be administered by any means suitable for the condition to be treated, which may depend on the need for site-specific treatment or quantity of drug to be delivered. Topical administration is generally preferred for skin-related diseases, and systematic treatment preferred for cancerous or pre-cancerous conditions, although other modes of delivery are contemplated. For example, the compounds of claim 1 may be delivered orally, such as in the form of tablets, capsules, granules, powders, or liquid formulations including syrups; topically, such as in the form of solutions, suspensions, gels or ointments; sublingually; bucally; parenterally, such as by subcutaneous, intravenous, intramuscular or intrasternal injection or infusion techniques (*e.g.*, as sterile injectable aq. or non-aq. solutions or suspensions); nasally such as by inhalation spray; topically, such as in the form of a cream or ointment; rectally such as in the form of suppositories; or liposomally. Dosage unit formulations containing non-toxic, pharmaceutically acceptable vehicles or diluents may be administered. The compound of claim 1, prepared according to the inventive process, may be administered in a form suitable for immediate release or extended release. Immediate release or extended release may be achieved with suitable pharmaceutical compositions or, particularly in the case of extended release, with devices such as subcutaneous implants or osmotic pumps.

Exemplary compositions for topical administration include a topical carrier such as PLASTIBASE® (mineral oil gelled with polyethylene).

Exemplary compositions for oral administration include suspensions which may contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which may contain, for example, microcrystalline cellulose, dicalcium phosphate, starch, magnesium stearate and/or lactose and/or other excipients, binders, extenders, disintegrants, diluents and lubricants such as those known in the art. The compound of claim 1 may also be orally delivered by sublingual and/or buccal administration, *e.g.*, with molded, compressed, or freeze-dried tablets. Exemplary compositions may include fast-dissolving diluents such as mannitol, lactose, sucrose, and/or cyclodextrins. Also included in such formulations may be high molecular weight excipients such as celluloses (AVICEL®) or polyethylene glycols (PEG); an excipient to aid mucosal adhesion such as hydroxypropyl cellulose (HPC), hydroxypropyl methyl cellulose (HPMC), sodium carboxymethyl cellulose (SCMC), and/or maleic anhydride copolymer (*e.g.*, GANTREZ®); and agents to control release such as polyacrylic copolymer (*e.g.*, CARBOPOL 934®). Lubricants, glidants, flavors, coloring agents and stabilizers may also be added for ease of fabrication and use.

An example of a composition for oral administration is the compound of claim 1, lactose monohydrate (intra-granular phase), microcrystalline cellulose(intra-granular phase), croscarmellose sodium(intra-granular phase), hydroxypropyl cellulose(intra-granular phase), microcrystalline cellulose (extra-granular phase), croscarmellose sodium (extra-granular phase), and magnesium stearate (extragranular phase).

Exemplary compositions for nasal aerosol or inhalation administration include solutions which may contain, for example, benzyl alcohol or other suitable preservatives, absorption promoters to enhance absorption and/or bioavailability, and/or other solubilizing or dispersing agents such as those known in the art.

Exemplary compositions for parenteral administration include injectable solutions or suspensions which may contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid.

Exemplary compositions for rectal administration include suppositories which may contain, for example, suitable non-irritating excipients, such as cocoa butter, synthetic glyceride esters or polyethylene glycols, which are solid at ordinary temperatures but liquefy and/or dissolve in the rectal cavity to release the drug.

The effective amount of the compound of claim 1 may be determined by one of ordinary skill in the art, and includes exemplary dosage amounts for a mammal of from about 0.05 to 100 mg/kg of body weight of active compound per day, which may be administered in a single dose or in the form of individual divided doses, such as from 1 to 4 times per day. It will be understood that the specific dose level and frequency of dosage for any particular subject may be varied and will depend upon a variety of factors, including the activity of the specific compound employed, the metabolic stability and length of action of that compound, the species, age, body weight, general health, sex and diet of the subject, the mode and time of administration, rate of excretion, drug combination, and severity of the particular condition. Preferred subjects for treatment include animals, most preferably mammalian species such as humans, and domestic animals such as dogs, cats, horses, and the like. Thus, when the term "patient" is used herein, this term is intended to include all subjects, most preferably mammalian species, that are affected by mediation of Src kinase levels.

When administered intravenously, the crystalline monohydrate of the compound of formula IV is administered using the formulations of the invention. In one embodiment, the compounds of the present invention, are administered by IV infusion over a period of from about 10 minutes to about 3 hours, preferably about 30 minutes to about 2 hours, more preferably about 45 minutes to 90 minutes, and most preferably about 1 hour. Typically, the compounds are administered intravenously in a dose of from about 0.5 mg/m² to 65 mg/m², preferably about 1 mg/m² to 50 mg/m², more preferably about 2.5 mg/m² to 30 mg/m², and most preferably about 25 mg/m².

One of ordinary skill in the art would readily know how to convert doses from mg/kg to mg/m2 given either or both the height and or weight of the patient (See, e.g., http://www.fda.gov/cder/cancer/animalframe.htm).

As discussed above, the crystalline monohydrate of the compound of formula IV can be administered orally, intravenously, or both. In particular, the methods of the invention encompass dosing protocols such as once a day for 2 to 10 days, preferably every 3 to 9 days, more preferably every 4 to 8 days and most preferably every 5 days. In one embodiment there is a period of 3 days to 5 weeks, alternatively 4 days to 4 weeks, or 5 days to 3 weeks, or 1 week to 2 weeks, in between cycles where there is no treatment. In another embodiment the crystalline forms of the compounds of formula IV can be administered orally, intravenously, or both, once a day for 3 days, with a period of 1 week to 3 weeks in between cycles where there is no treatment. In yet another embodiment the compounds of the present invention, the crystalline forms of the compounds of formula IV, can be administered orally, intravenously, or both, once a day for 5 days, with a period of 1 week to 3 weeks in between cycles where there is no treatment.

In another embodiment the treatment cycle for administration of the compounds of the present invention, the crystalline monohydrate of the compound of formula IV, is once daily for 5 consecutive days and the period between treatment cycles is from 2 to 10 days, or alternatively one week. In one embodiment, a compound of claim 1, is administered once daily for 5 consecutive days, followed by 2 days when there is no treatment.

The compound of the present invention, the crystalline monohydrate of the compound of formula IV, can also be administered orally, intravenously, or both once every 1 to 10 weeks, every 2 to 8 weeks, every 3 to 6 weeks, alternatively every 3 weeks.

In another method of the invention, the compound of the present invention, the crystalline monohydrate of the compound of formula IV, are administered in a 28 day cycle wherein the compound is intravenously administered on days 1, 7, and 14 and orally administered on day 21. Alternatively, the compound of the present invention, the crystalline monohydrate of the compound of formula IV, are administered in a 28 day cycle wherein the compound of formula IV is orally administered on day 1 and intravenously administered on days 7,14, and 28.

According to the methods of administering the crystalline compound of the invention, the compound of formulae IV is administered until the patient shows a response, for example, a reduction in tumor size, or until dose limiting toxicity is reached.

The Compound within the scope of the invention may be tested for activity as inhibitors of protein kinases using the assays described below, or variations thereof that are within the level ordinary skill in the art.

### Cell assays

### (1) Cellular tyrosine phosphorylation

Jurkat T cells are incubated with the test compound and then stimulated by the addition of antibody to CD3 (monoclonal antibody G19-4). Cells are lysed after 4 minutes or at another desired time by the addition of a lysis buffer containing NP-40 detergent. Phosphorylation of proteins is detected by anti-phosphotyrosine immunoblotting. Detection of phosphorylation of specific proteins of interest such as ZAP-70 is detected by immunoprecipitation with anti-ZAP-70 antibody followed by anti-phosphotyrosine immunoblotting. Such procedures are described in Schieven, G.L., Mittler, RS., Nadler, S.G., Kirihara, J.M., Bolen, J.B., Kanner, S.B., and Ledbetter, J.A., "ZAP-70 tyrosine kinase, CD45 and T cell receptor involvement in UV and H2O2 induced T cell signal transduction", J. Biol. Chem., 269, 20718-20726 (1994), and the references incorporated therein. The Lck inhibitors inhibit the tyrosine phosphorylation of cellular proteins induced by anti-CD3 antibodies.

For the preparation of G19-4, see Hansen, J.A., Martin, P.J., Beatty, P.G., Clark, E.A., and Ledbetter, J.A., "Human T lymphocyte cell surface molecules defined by the workshop monoclonal antibodies," in Leukocyte Typing I, A. Bernard, J. Boumsell, J. Dausett, C. Milstein, and S. Schlossman, eds. (New York: Springer Verlag), p. 195-212 (1984); and Ledbetter, J.A., June, C.H., Rabinovitch, P.S., Grossman, A., Tsu, T.T., and Imboden, J.B., "Signal transduction through CD4 receptors: stimulatory vs. inhibitory activity is regulated by CD4 proximity to the CD3/T cell receptor", Eur. J. Immunol., 18, 525 (1988).

### (2) Calcium assay

Lck inhibitors block calcium mobilization in T cells stimulated with anti-CD3 antibodies. Cells are loaded with the calcium indicator dye indo-1, treated with anti-CD3 antibody such as the monoclonal antibody G19-4, and calcium mobilization is measured using flow cytometry by recording changes in the blue/violet indo-1 ratio as described in Schieven, G.L., Mittler, R.S., Nadler, S.G., Kirihara, J.M., Bolen, J.B., Kanner, S.B., and Ledbetter, J.A., "ZAP-70 tyrosine kinase, CD45 and T cell receptor involvement in UV and H2O2 induced T cell signal transduction", J. Biol. Chem., 269, 20718-20726 (1994), and the references incorporated therein.

### (3) Proliferation assays

Lck inhibitors inhibit the proliferation of normal human peripheral blood T cells stimulated to grow with anti-CD3 plus anti-CD28 antibodies. A 96 well plate is coated with a monoclonal antibody to CD3 (such as G19-4), the antibody is allowed to bind, and then the plate is washed. The antibody bound to the plate serves to stimulate the cells. Normal human peripheral blood T cells are added to the wells along with test compound plus anti-CD28 antibody to provide co-stimulation. After a desired period of time (*e.g.*, 3 days), the [3H]-thymidine is added to the cells, and after further incubation to allow incorporation of the label into newly synthesized DNA, the cells are harvested and counted in a scintillation counter to measure cell proliferation.

The following examples illustrate the invention but should not be interpreted as a limitation thereon.

### EXAMPLES

### Example 5

### Preparation of:

### N-(2-chloro-6-methylphenyl)-2-(6-(4-(3-hydroxyethyl)piperazin-1-yl)-2-methylpyrimidin-4-ylamino)thiazole-5-carboxamide (The compound of Formula (IV))

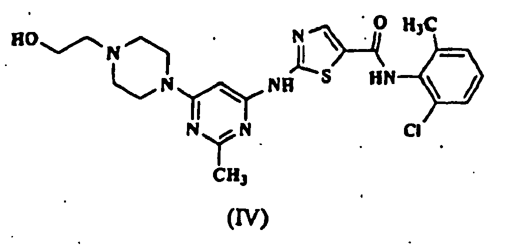

### 5A. 1-(6-Chloro-2-methylpyrimidin-4-yl)thiourea

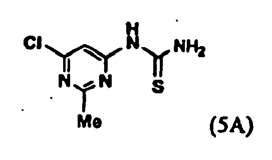

To a stirring slurry of 4-amino-5-chloro-2-methylpyrimidine (6.13g, 42.7 mmol) in THF (24 mL) was added ethyl isothiocyanatoformate (7.5 mL, 63.6 mmol), and the mixture heated to reflux. After 5h, another portion of ethyl isothiocyanato formate (1.0 mL, 8.5 mmol) was added and after 10h, a final portion (1.5 mL, 12.7 mmol) was added and the mixture stirred 6h more. The slurry was evaporated under vacuum to remove most of the solvent and heptane (6 mL) added to the residue. The solid was collected by vacuum filtration and washed with heptane (2 x 5 mL) giving 8.01 g (68 % yield) of the intermediate ethyl 6-chloro-2-methylpyrimidin-4-ylcarbamothioylcarbamate.

A solution of ethyl 6-chloro-2-methylpyrimidin-4-ylcarbamothioylcarbamate (275 mg, 1.0 mmol) and 1N sodium hydroxide (3.5eq) was heated and stirred at 50°C for 2h. The resulting slurry was cooled to 20-22°C. The solid was collected by vacuum filtration, washed with water, and dried to give 185 mg of 1-(6-chloro-2-methylpyrimidin-4-yl)thiourea (91% yield). ¹H NMR (400MHz, DMSO-d₆): δ2.51 (S, 3H), 7.05 (s, 1H), 9.35 (s,1H), 10.07 (s, 1H), 10.91 (s, 1H); ¹³C NMR (125MHz, DMSO-d6) δ: 25.25, 104.56, 159.19, 159.33,167.36, 180.91.

### 5B. (E)-N-(2-Chloro-6-methylphenyl)-3-ethoxyacrylamide

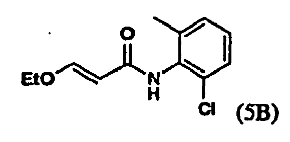

To a cold stirring solution of 2-chloro-6-methylaniline (59.5 g 0.42 mol) and pyridine (68 ml, 0.63 mol) in THF (600 mL) was added 3-ethoxyacryloyl chloride (84.7 g, 0.63 mol) slowly keeping the temp at 0-5°C. The mixture was then warmed and stirred for 2 h. at 20°C. Hydrochloric acid (1N, 115 mL) was added at 0-10°C. The mixture was diluted with water (310 mL) and the resulting solution was concentrated under vacuum to a thick slurry. The slurry was diluted with toluene (275 mL) and stirred for 15 min. at 20-22°C then 1 h. at 0°C. The solid was collected by vacuum filtration, washed with water (2 x 75 mL) and dried to give 74.1 g (73.6 % yield) of (E)-N-(2-chloro-6-methylphenyl)-3-ethoxyacrylamide). ¹H NMR (400 Hz, DMSO-d₆) δ1.26 (t, 3H, J= 7 Hz), 2.15 (s, 3H), 3.94 (q, 2H, J= 7 Hz), 5.58 (d, 1H, J=12.4 Hz), 7.10-7.27 (m, 2H, J=7.5 Hz), 7.27-7.37 (d, 1H, J=7.5 Hz), 7.45(d, 1H, J=12^{.}4 Hz), 9.28 (s, 1H); ¹³C NMR (100MHz, CDCl₃) δ: 14.57,18.96,67.17,97.99, 126.80, 127.44, 129.07, 131.32, 132.89, 138.25, 161.09, 165.36.

### 5C. 2-Amino-N-(2-chloro-6-methylphenyl)thiazole-5-carboxamide

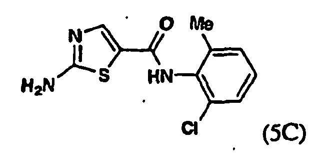

To a mixture of compound **5B** (5.00 g, 20.86 mmol) in 1,4-dioxane (27 mL) and water (27 mL) was added NBS (4.08 g, 22.9 mmol) at -10 to 0°C. The slurry was warmed and stirred at 20-22°C for 3h. Thiourea (1.60 g, 21 mmol) was added and the mixture heated to 80°C. After 2h, the resulting solution was cooled to 20-22° and conc. ammonium hydroxide (4.2 mL) was added dropwise. The resulting slurry was concentrated under vacuum to about half volume and cooled to 0-5°C. The solid was collected by vacuum filtration, washed with cold water (10 mL), and dried to give 5.3 g (94.9 % yield) of 2-amino-N-(2-chloro-6-methylphenyl)thiazole-5-carboxamide. ¹H NMR (400 MHz, DMSO-d₆) δ δ 2.19 (s, 3H), 7.09-7.29 (m, 2H, J=7.5), 7.29-7.43 (d, 1H, J=7.5), 7.61 (s, 2H), 7.85 (s, 1H), 9.63 (s, 1H); ¹³C NMR (125MHz, DMSO-d6) δ: 18.18, 120.63, 126.84, 127.90, 128.86, 132.41, 133.63, 138.76, 142.88, 159.45, 172.02.

### 5D. 2-(6-Chloro-2-methylpyrimidin-4-ylamino)-N-(2-chloro-6-methylphenyl)thiazole-5-carboxamide

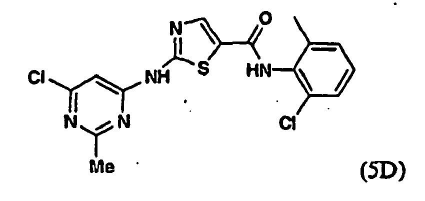

To a stirring solution of compound **5C** (5.00 g, 18.67 mmol) and 4,6-dichloro-2-methylpyrimidine (3.65 g 22.4/mmol) in THF (65 mL) was added a 30% wt. solution of sodium t-butoxide in THF (21.1g, 65.36 mmol) slowly with cooling to keep the temperature at 10-20°C. The mixture was stirred at room temperature for 1.5 h and cooled to 0-5°C. Hydrochloric acid, 2N (21.5 mL) was added slowly and the mixture stirred 1.75 h at 0-5°C. The solid was collected by vacuum filtration, washed with water (15 mL) and dried to give 6.63 g (86.4 % yield) of compound **5D**. ¹H NMR (400 MHz, DMSO-d₆) δ 2.23 (s, 3H), 2.58 (s, 3H), 6.94 (s, 1H), 7.18-7.34, ( m, 2H, J=7.5), 7.34-7.46 (d, 1H, , J=7.5), 8.31 (s, 1H), 10.02 (s, 1H), 12.25 (s, 1H).

### 5E. Example 5

To a mixture of compound **5D** (4.00 g, 10.14 mmol) and hydroxyethylpiperazine (6.60 g, 50.69 mmol) in n-butanol (40 mL) was added DIPEA (3.53 mL, 20.26 mmol). The slurry was heated at 118°C for 4.5h, then cooled slowly to room temperature. The solid was collected by vacuum filtration, washed with n-butanol (5 mL), and dried. The product (5.11 g) was dissolved in hot 80% EtOH-H₂O (80 mL), and the solution was clarified by filtration. The hot solution was slowly diluted with water (15 mL) and cooled slowly to room temperature. The solid was collected by vacuum filtration, washed with 50% ethanol-water (5 mL) and dried affording 4.27 g (83.2 % yield) of N-(2-chloro-6-methylphenyl)-2-(6-(4-(3-hydroxyethyl)piperazin-1-yl)-2-methylpyrimidin-4-ylamino)thiazole-5-carboxamide as monohydrate. ¹H NMR (400 MHz, DMSO-d₆) δ 2.23 (s, 3H), 2.40 (s, 3H), 2.42 (t, 2H, J=6), 2.48 (t, 4H, J=6.3), 3.50 (m, 4H), 3.53 (q, 2H, J=6), 4.45 (t, 1H, J=5.3), 6.04 (s, 1H), 7.25 (t, 1H, J=7.6), 7.27 (dd, 1H, J=7.6, 1.7), 7.40 (dd, 1H, J=7.6, 1.7), 8.21 (s, 1H), 9.87 (s, 1H), 11.47.

### EXAMPLE 6

### Preparation of:

### N-(2-chloro-6-methylphenyl)-2-(6-(4-(3-hydroxyethyl)piperazin-1-yl)-2-methylpyrimidin-4-ylamino)thiazole-5-carboxamide

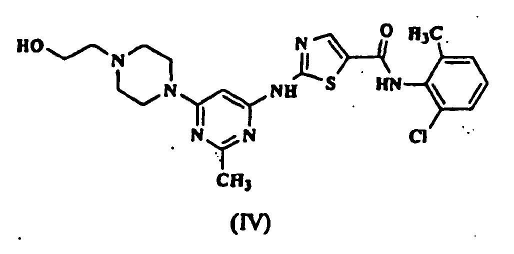

To a slurry of (E)-N-(2-chloro-6-methylphenyl)-3-ethoxyacrylamide **5B** (120 mg, 0.50 mmol) in THF (0.75 ml) and water (0.5 mL) was added NBS (98 mg, 0.55 mmol) at 0°C. The mixture was warmed and stirred at 20-22°C for 3h. To this was added 1-(6-chloro-2-methylpyrimidin-4-yl)thiourea **5A** (100 mg, 0.49 mmol), and the slurry heated and stirred at reflux for 2h. The slurry was cooled to 20-22°C. and the solid collected by vacuum filtration giving 140 mg (71% yield) of 2-(6-chloro-2-methylpyrimidin-4-ylamino)-N-(2-chloro-6-methylphenyl)thiazole-5-carboxamide **5D**. ¹H NMR (400 MHz, DMSO-d₆) δ 2.23 (s, 3H), 2.58 (s, 3H), 6.94 (s, 1H), 7.18-7.34, ( m, 2H, J=7.5), 7.34-7.46 (d, 1H, , J=7.5), 8.31 (s, 1H), 10.02 (s, 1H), 12.25 (s, 1H).

Compound **5D** was elaborated to N-(2-chloro-6-methylphenyl)-2-(6-(4-(3-hydroxyethyl)piperazin-1-yl)-2-methylpyrimidin-4-ylamino)thiazole-5-carboxamide, following Step **5E**.

### ANALYTICAL METHODS

### Solid State Nuclear Magnetic Resonance (SSNMR)

All solid-state C-13 NMR measurements were made with a Bruker DSX-400, 400 MHz NMR spectrometer. High resolution spectra were obtained using high-power proton decoupling and the TPPM pulse sequence and ramp amplitude cross-polarization (RAMP-CP) with magic-angle spinning (MAS) at approximately 12 kHz (A.E. Bennett et al, J. Chem. Phys.,1995, 103, 6951), (G, Metz, X Wu and S.O. Smith, J. Magn. Reson, A,. 1994, 110, 219-227). Approximately 70 mg of sample, packed into a canister-design zirconia motor was used for each experiment. Chemical shifts (δ) were referenced to external adamantane with the high frequency resonance being set to 38.56 ppm (W.L. Earl and D.L. VanderHart, J. Magn. Reson., 1982, 48, 35-54).

### x-Ray Powder Diffraction

One of ordinary skill in the art will appreciate that an X-ray diffraction pattern may be obtained with a measurement error that is dependent upon the measurement conditions employed. In particular, it is generally known that intensities in a X-xay diffraction pattern may fluctuate depending upon measurement conditions employed. It should be further understood that relative intensities may also vary depending upon experimental conditions and, accordingly, the exact order of intensity should not be taken into account. Additionally, a measurement error of diffraction angle for a conventional X-ray diffraction pattern is typically about 5% or less, and such degree of measurement error should be taken into account as pertaining to the aforementioned diffraction angles. Consequently, it is to be understood that the crystal forms of the instant invention are not limited to the crystal forms that provide X-ray diffraction patterns completely identical to the X-ray diffraction patterns depicted in the accompanying Figures disclosed herein. Any crystal forms that provide X- ray diffraction patterns substantially identical to those disclosed in the accompanying Figures fall within the scope of the present invention. The ability to ascertain substantial identities of X-ray diffraction patterns is within the purview of one of ordinary skill in the art.

X-Ray powder diffraction data for the crystalline forms of compound (IV) were obtained using a Bruker GADDS (**BRUKER AXS**, Inc., 5465 East Cheryl Parkway Madison, WI 53711 USA) (General Area Detector Diffraction System) manual chi platform goniometer. Powder samples were placed in thin walled glass capillaries of 1mm or less in diameter; the capillary was rotated during data collection. The sample-detector distance was 17 cm. The radiation was Cu Kα (45kV 111mA, λ, = 1.5418 Å). Data were collected for 3<2θ <35° with a sample exposure time of at least 300 seconds.

### Single Crystal X-Ray

All single crystal data were collected on a Bruker-Nonius (**BRUKER AXS, Inc.**, 5465 East Cheryl Parkway Madison, WI 53711 USA) Kappa CCD 2000 system using Cu Kα radiation (λ, = 1.5418 Å) and were corrected only for the Lorentz-polarization factors. Indexing and processing of the measured intensity data were carried out with the HKL2000 software package (Otwinowski, Z. & Minor, W. (1997) in Macromolecular Crystallography, eds. Carter, W.C. Jr & Sweet, R.M. (Academic, NY), Vol. 276, pp.307-326) in the Collect program suite (Data collection and processing user interface: Collect: Data collection software, R. Hooft, Nonius B.V., 1998).

The structures were .solved by direct methods and refined on the basis of observed reflections using either the SDP (SDP, Structure Determination Package,Enraf-Nonius, Bohemia NY 11716 Scattering factors, including *f'*and *f"*, in the SDP software were taken from the" International Tables for Crystallography", Kynoch Press, Birmingham, England, 1974; Vol IV, Tables 2.2A and 2.3.1) software package with minor local modifications or the crystallographic package, MAXUS (maXus solution and refinement software suite: S. Mackay, C.J. Gilmore, C. Edwards, M. Tremayne, N. Stewart, K. Shankland, maXus: a computer program for the solution and refinement of crystal structures from diffraction data).

The derived atomic parameters (coordinates and temperature factors) were refined through full matrix least-squares. The function minimized in the refinements was Σ_{w}(|Fo| -|F_{c}|)^{2.} R is defined as Σ∥Fₒ|-|F_{c}∥/Σ|Fₒ| while R_{w} = [F_{w}(∥Fₒ|-|F_{c}|)₂/Σ_{w} |Fₒ|²]^{1/2} where w is an appropriate weighting function based on errors in the observed intensities. Difference maps were examined at all stages of refinement. Hydrogens were introduced in idealized positions with isotropic temperature factors, but no hydrogen parameters were varied.

The derived atomic parameters (coordinates and temperature factors) were refined through full matrix least-squares. The function minimized in the refinements was Σ_{w}(|Fₒ| - |F_{c}|)^{2.} R is defined as Σ ∥Fₒ|- |F_{c}∥/Σ|Fₒ| while R_{w} = [Σ_{w}(|Fₒ| - |F_{c}|)₂/Σ_{w} |Fₒ|²]^{1/2} where w is an appropriate weighting function based on errors in the observed intensities. Difference maps were examined at all stages of refinement. Hydrogens were introduced in idealized positions with isotropic temperature factors, but no hydrogen parameters were varied

### Differential Scanning Calorimetry

The DSC instrument used to test the crystalline forms was a TA Instruments® model Q1000. The DSC cell/sample chamber was purged with 100 ml/min of ultrahigh purity nitrogen gas. The instrument was calibrated with high purity indium. The accuracy of the measured sample temperature with this method is within about +/-1°C, and the heat of fusion can be measured within a relative error of about +/-5%. The sample was placed into an open aluminum DSC pan and measured against an empty reference pan. At least 2 mg of sample powder was placed into the bottom of the pan and lightly tapped down to ensure good contact with the pan. The weight of the sample was measured accurately and recorded to a hundredth of a milligram. The instrument was programmed to heat at 10°C per minute in the temperature range between 25 and 350°C.

The heat flow, which was normalized by a sample weight, was plotted versus the measured sample temperature. The data were reported in units of watts/gram ("W/g"). The plot was made with the endothermic peaks pointing down. The endothermic melt peak was evaluated for extrapolated onset temperature, peak temperature, and heat of fusion in this analysis.

### Thermogravimetric Analysis (TGA)

The TGA instrument used to test the crystalline forms was a TAInstruments® model Q500. Samples of at least 10 milligrams were analyzed at a heating rate of 10°C per minute in the temperature range between 25°C and about 350°C.

### EXAMPLE 8

### Preparation of:

### crystalline monohydrate of N-(2-chloro-6-methylphenyl)-2-(6-(4-(3-hydroxyethyl)piperazin-1-yl)-2-methylpyrimidin-4-ylamino)thiazole-5-carboxamide (IV)

An example of the crystallization procedure to obtain the crystalline monohydrate form is shown here:
Charge 48 g of the compound of formula (IV).
Charge approximately 1056 mL (22 mL/g) of ethyl alcohol, or other suitable alcohol.
Charge approximately 144 mL of water.
Dissolve the suspension by heating to approximately 75 °C.
Optional: Polish filter by transfer the compound of formula (IV) solution at 75 °C through the preheated filter and into the receiver.
Rinse the dissolution reactor and transfer lines with a mixture of 43 mL of ethanol and 5 mL of water.

Heat the contents in the receiver to 75 - 80 °C and maintain 75 - 80 °C to achieve complete dissolution.

Charge approximately 384 mL of water at a rate such that the batch temperature is maintained between 75-80 °C.

Cool to 75 °C; and, optionally, charge monohydrate seed crystals. Seed crystals are not essential to obtaining monohydrate, but provide better control of the crystallization.
Cool to 70 °C and maintain 70 °C for ca. 1 h.
Cool from 70 to 5 C over 2 h, and maintain the temperature between 0 at 5 °C for at least 2 h.
Filter the crystal slurry.
Wash the filter cake with a mixture of 96 mL of ethanol and 96 mL of water.
Dry the material at ≤ 50 °C under reduced pressure until the water content is 3.4 to 4.1 % by KF to afford 41 g (85 M%).

Alternately, the monohydrate can be obtained by:
1) An aqueous solution of the acetate salt of compound IV was seeded with monohydrate and heated at 80 °C to give bulk monohydrate.
2) An aqueous solution of the acetate salt of compound IV was seeded with monohydrate. On standing several days at room temperature, bulk monohydrate had formed.
3) An aqueous suspension of compound IV was seeded with monohydrate and heated at 70 °C for 4 hours to give bulk monohydrate. In the absence of seeding, an aqueous slurry of compound IV was unchanged after 82 days at room temperature.
4) A solution of compound IV in a solvent such as NMP or DMA was treated with water until the solution became cloudy and was held at 75-85 °C for several hours. Monohydrate was isolated after cooling and filtering.
5)A solution of compound IV in ethanol, butanol, and water was heated. Seeds of monohydrate were added to the hot solution and then cooled. Monohydrate was isolated upon cooling and filtration.

One of ordinary skill in the art will appreciate that the monohydrate of the compound of formula (IV) may be represented by the XRPD as shown in Figure 1 or by a representative sampling of peaks as shown in Table 1.

Representative peaks taken from the XRPD of the monohydrate of the compound of formula (IV) are shown in Table 1.

**Table 1.**

| 2-Theta | d(Å) | Height |
|---|---|---|
| 17.994 | 4.9257 | 915 |
| 18.440 | 4.8075 | 338 |
| 19.153 | 4.6301 | 644 |
| 19.599 | 4.5258 | 361 |
| 21.252 | 4.1774 | 148 |
| 24.462 | 3.6359 | 250 |
| 25.901 | 3.4371 | 133 |
| 28.052 | 3.1782 | 153 |

The XRPD is also characterized by the following list comprising 2θ values selected from the group consisting of: 4.6± 0.2,11.2± 0.2,13.8± 0.2,15.2± 0.2, 17.9± 0.2, 19.1± 0.2,19.6± 0.2, 23.2± 0.2, 23.6± 0.2. The XRPD is also characterized by the list of 2θ values selected from the group consisting of: 18.0± 0.2, 18.4± 0.2, 19.2± 0.2,19.6± 0.2, 21.2± 0.2, 24.5± 0.2, 25.9± 0.2, and 28.0± 0.2.

Single crystal x-ray data was obtained at room temperature (+25°C). The molecular structure was confirmed as a monohydrate form of the compound of Formula (IV).

The following unit cell parameters were obtained for the monohydrate of the compound of formula (IV) from the x-ray analysis at 25°C:
a(Å) =13.8632(7); b(Å)= 9.3307(3); c(A) = 38.390(2);
V(Å³) 4965.9(4); Z' = 1; Vm = 621
Space group Pbca
Molecules/unit cell 8
Density (calculated) (g/cm³) 1.354
   Wherein Z' = number of drug molecules per asymmetric unit. Vm = V(ᵤₙᵢₜ cell) / (Z drug molecules per cell).

Single crystal x-ray data was also obtained at -50°C. The monohydrate form of the compound of Formula (IV) is characterized by unit cell parameters approximately equal to the following:
Cell dimensions: a(Å) = 13.862(1);
b(Å)= 9.286(1);
c(Å)= 38.143(2);
Volume = 4910(1) Å³
Space group Pbca
Molecules/unit cell 8
Density (calculated) (g/cm³) 1.300
   wherein the compound is at a temperature of about -50°C.

The simulated XRPD was calculated from the refined atomic parameters at room temperature.

The monohydrate of the compound of formula (IV) is represented by the DSC as shown in Figure 2. The DSC is characterized by a broad peak between approximately 95°C and 130°C. This peak is broad and variable and corresponds to the loss of one water of hydration as seen in the TGA graph. The DSC also has a characteristic peak at approximately 287°C which corresponds to the melt of the dehydrated form of the compound of formula (IV).

The TGA for the monohydrate of the compound of Formula (IV) is shown in Figure 2 along with the DSC. The TGA shows a 3.48% weight loss from 50°C to 175°C. The weight loss corresponds to a loss of one water of hydration from the compound of Formula (IV).

The monohydrate , may also be prepared by crystallizing from alcoholic solvents, such as methanol, ethanol, propanol, i-propanol, butanol, pentanol, and water.

### EXAMPLE 9

### Preparation of:

### crystalline n-butanol solvate of N-(2-chloro-6-methylphenyl)-2-(6-(4-(3-hydroxyethyl)piperazin-1yl)-2-methylpyrimidin-4-ylamino)thiazole-5-carboxamide

The crystalline butanol solvate of the compound of formula (IV) is prepared by dissolving compound (IV) in 1-butanol at reflux (1116-118°C) at a concentration of approximately 1g/25 mL of solvent. Upon cooling, the butanol solvate crystallizes out of solution. Filter, wash with butanol, and dry.

The following unit cell parameters were obtained from the x-ray analysis for the crystalline butanol solvate, obtained at room temperature:
a(Å) = 22.8102(6); b(Å) = 8.4691 (3); c(Å) = 15.1436(5);
V(Å) 2910.5(2); Z' = 1; Vm = 728
Space group P2₁/a
Molecules/unit cell 4
Density (calculated) (g/cm³) 1.283
   Wherein Z' = number of drug molecules per asymmetric unit. Vm = V(unit cell) / (Z drug molecules per cell).

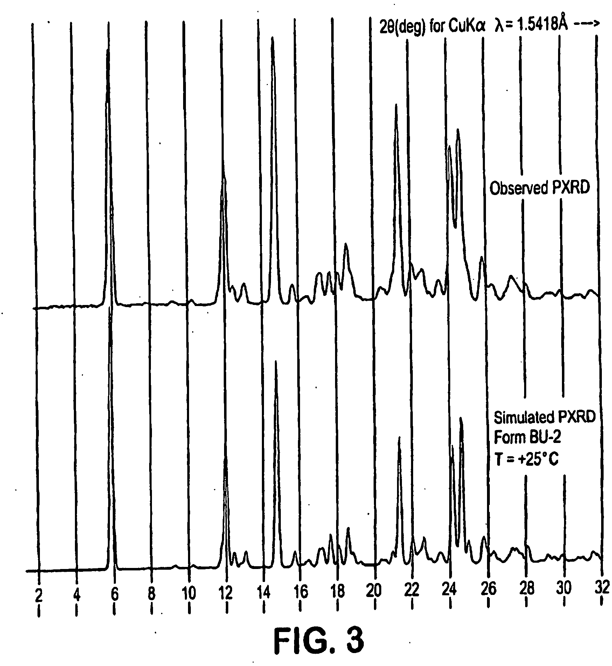

One of ordinary skill in the art will appreciate that the butanol solvate of the compound of formula (IV) may be represented by the XRPD as shown in Figure 3 or by a representative sampling of peaks. Representative peaks for the crystalline butanol solvate are 20 values of : 5.9 ± 0.2, 12.0 ± 0.2, 13.0 ± 0.2, 17.7 ± 0.2, 24.1 ± 0.2, and 24.6 ±0.2.

## Claims

1. Crystalline monohydrate of the compound of formula (IV)

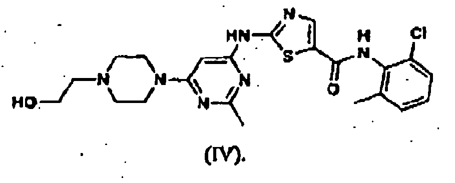

2. The compound of Claim 1, which is **characterized by** an x-ray powder diffraction pattern (CuKα λ=1.5418Å at a temperature of about 23°C) comprising four or more 2θ values selected from the group consisting of: 18.0± 0.2,18.4± 0.2, 19.2± 0.2, 19.6± 0.2, 21.2± 0.2, 24.5± 0.2, 25.9± 0.2, and 28.0± 0.2.

3. The compound of claim 2, which is **characterized by** an x-ray powder diffraction pattern in accordance with that shown in Figure 1.

4. The compound of claim 1, **characterized by** unit cell parameters approximately equal to the following:
Cell dimensions: a(Å) =13.8632(7);
b(Å)= 9.3307(3);
c(Å) = 38.390(2);
Volume = 4965.9(4) Å³
Space group Pbca
Molecules/unit cell 8
Density (calculated) (g/cm³) 1.354.

5. The compound of claim 1, wherein there is one water molecule per molecule of formula (IV).

6. A pharmaceutical composition comprising a therapeutically effective amount of the compound of any one of claims 1 to 5 and a pharmaceutically acceptable carrier.

7. Use of a compound of any of claims 1 to 5 for the manufacture of a medicament for use in the treatment of cancer.

8. Use of a compound of any one of claims 1 to 5 for the manufacture of a medicament for use in treating oncological disorders wherein the disorders are selected from chronic myelogenous leukemia (CML), gastrointestinal stromal tumor (GIST), small cell lung cancer (SCLC), non-small cell lung cancer (NSCLC), ovarian cancer, melanoma, mastocytosis, germ cell tumors, acute myelogenous leukemia (AML), pediatric sarcomas, breast cancer, colorectal cancer, pancreatic cancer, and prostate cancer.

9. A compound of any one of claims 1 to 5 for use in therapy.

10. A process for preparing crystalline monohydrate of the compound of formula (IV) 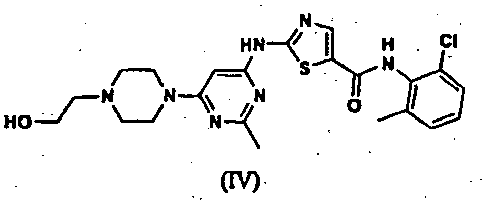 comprising heating and dissolving the compound of formula (IV) in an ethanol/water mixture and crystallizing the monohydrate from the ethanol/water mixture as it cools.

11. The process of claim 10, wherein the butanol solvate of the compound of formula (IV) is dissolved in the ethanol/water mixture.

## Patentansprüche

1. Kristallines Monohydrat der Verbindung der Formel (IV)

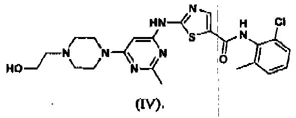

2. Verbindung nach Anspruch 1, welche durch ein Röntgenpulverbeugungsdiagramm (CuKα λ = 1,5418 Å bei einer Temperatur von etwa 23°C), umfassend vier oder mehr 2θ-Werte, ausgewählt aus 18,0 ± 0,2, 18,4 ± 0,2, 19,2 ± 0,2, 19,6 ± 0.2, 21,2 ± 0.2, 24,5 ± 0,2, 25,9 ± 0,2 und 28,0 ± 0,2 gekennzeichnet ist.

3. Verbindung nach Anspruch 2, welche durch ein Röntgenpulverbeugungsdiagramm in Übereinstimmung mit dem, gezeigt in Figur 1, gekennzeichnet ist.

4. Verbindung nach Anspruch 1, **gekennzeichnet durch** Parameter der Elementarzelle ungefähr gleich den folgenden:
| | |
|---|---|
| Zellabmessungen: | a (Å) = 13,8632(7); |
| | b (Å) = 9,3307(3); |
| | c (Å) = 38,390(2); |
| Volumen = 4965,9(4) Å³ | |
| Raumgruppe Pbca | |
| Moleküle/Elementarzelle | 8 |
| Dichte (berechnet) (g/cm³) | 1,354. |

5. Verbindung nach Anspruch 1, wobei es ein Wassermolekül pro Molekül der Formel (IV) gibt.

6. Pharmazeutische Zusammensetzung, umfassend eine therapeutisch wirksame Menge der Verbindung nach einem der Ansprüche 1 bis 5 und einen pharmazeutisch verträglichen Träger.

7. Verwendung einer Verbindung nach einem der Ansprüche 1 bis 5 für die Herstellung eines Medikaments zur Verwendung in der Behandlung von Krebs.

8. Verwendung einer Verbindung nach einem der Ansprüche 1 bis 5 für die Herstellung eines Medikaments zur Verwendung beim Behandeln von onkologischen Krankheiten, wobei die Krankheiten aus chronischer myelogener Leukämie (CML), gastrointestinalem Stromatumor (GIST), kleinzelligern Lungenkrebs (SCLC), nichtkleinzelligern Lungenkrebs (NSCLC), Eierstockkrebs, Melanom, Mastozytose, Keimzelltumoren, akuter myelogener Leukämie (AML), pädiatrischen Sarkomen, Brustkrebs, kolorektalem Krebs, Bauchspeicheldrüsenkrebs und Prostatakrebs ausgewählt sind.

9. Verbindung nach einem der Ansprüche 1 bis 5 zur Verwendung in der Therapie.

10. Verfahren zum Herstellen von kristallinem Monohydrat der Verbindung der Formel (IV) 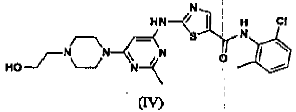 umfassend Erhitzen und Lösen der Verbindung der Formel (IV) in einem Ethanol/Wasser-Gernisch und Kristallisieren des Monohydrats aus dem Ethanol/WasserGemisch, wenn es abkühft.

11. Verfahren nach Anspruch 10, wobei das Butanolsolvat der Verbindung der Formel (IV) in dem Ethanol/Wasser-Gernisch gelöst wird.

## Revendications

1. Monohydrate cristallin du composé de la formule (IV)

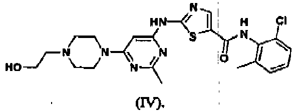

2. Le composé de la revendication 1 qui est **caractérisé par** un réseau de diffraction des rayons x sur poudre (CuKα λ = 1,5418 Å à une température d'environ 23°C) comprenant quatre valeurs 2θ ou plus sélectionnées parmi le groupe consistant en: 18,0 ± 0,2, 18,4 ± 0.2, 19,2 ± 0,2, 19,6 ± 0.2, 21,2 ± 0,2, 24,5 ± 0,2, 25,9 ± 0,2 et 28,0 ± 0,2.

3. Le composé de la revendication 2 qui est **caractérisé par** un réseau de diffraction des rayons x sur poudre conformément à celui qui est illustré sur la Figure 1.

4. Le composé de la revendication 1, **caractérisé par** des paramètres de cellule unitaire approximativement égaux à ce qui suit:
| | |
|---|---|
| Dimensions des cellules: | a (Å) = 13,8632(7); |
| | b (Å) = 9,3307(3); |
| | c (Å) = 38,390(2); |
| Volume = 4965,9(4) Å³ | |
| Groupe spatial Pbca | |
| Molécule/cellule unitaire | 8 |
| Densité (calculée) (g/cm³) | 1,354. |

5. Le composé de la revendication 1, dans lequel il y a une molécule d'eau par molécule de la formule (IV).

6. Composition pharmaceutique comprenant une quantité thérapeutiquement efficace du composé de l'une quelconque des revendications 1 à 5 et un excipient pharmaceutiquement acceptable.

7. Utilisation d'un composé de l'une quelconque des revendications 1 à 5 dans la fabrication d'un médicament à utiliser dans le traitement du cancer.

8. Utilisation d'un composé de l'une quelconque des revendications 1 à 5 dans la fabrication d'un médicament à utiliser dans le traitement de troubles oncologiques où les troubles sont sélectionnés parmi la leucémie myéloïde chronique (LMC), les tumeurs stromales gastro-intestinales (GIST), le cancer du poumon à petites cellules (SCLC), le cancer du poumon non à petites cellules (NSCLC), le cancer de l'ovaire, le mélanome, la mastocytose, les tumeurs de cellules germinales, la leucémie myéloïde aiguë (LMA), les sarcomes pédiatriques, le cancer du sein, le cancer colorectal, le cancer du pancréas et le cancer de la prostate.

9. Composé de l'une quelconque des revendications 1 à 5 à utiliser en thérapie.

10. Procédé de préparation de monohydrate cristallin du composé de la formule (IV) 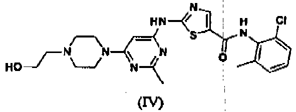 comprenant chauffer et dissoudre le composé de la formule (IV) dans un mélange d'éthanol/eau et cristalliser le monohydrate du mélange d'éthanol/eau au fur et à mesure qu'il refroidit.

11. Le procédé de la revendication 10, dans lequel le solvate de butanol du composé de la formule (IV) est dissous dans le mélange d'éthanol/eau.
